Europäisches Patentamt

European Patent Office

Office européen des brevets

(11) Publication number : **0 140 865**
**B1**

# (12) EUROPEAN PATENT SPECIFICATION

(45) Date of publication of patent specification :
25.03.87

(21) Application number : 84870126.4

(22) Date of filing : 04.09.84

(51) Int. Cl.⁴ : **C 07 C149/20**, C 07 C148/00//
A23K1/16

(54) Enhanced 2-hydroxy-4-methylthiobutanoic acid composition and method of preparation.

(30) Priority : 06.09.83 US 529191

(43) Date of publication of application :
08.05.85 Bulletin 85/19

(45) Publication of the grant of the patent :
25.03.87 Bulletin 87/13

(84) Designated contracting states :
BE DE FR GB NL

(56) References cited :
EP-A- 0 049 057
DE-A- 2 945 497
DE-B- 1 133 366
DE-B- 1 157 216
US-A- 3 272 860
US-A- 4 310 690

(73) Proprietor : **Monsanto Company**
**Patent Department 800 North Lindbergh Boulevard**
**St. Louis Missouri 63167 (US)**

(72) Inventor : **Takano, Masaharu**
**13146 Roundstone Court**
**St. Louis, MO 63146 (US)**

(74) Representative : **Lunt, John Cooper et al**
**Monsanto Europe S.A. Patent Department Avenue de**
**Tervuren 270-272 Letter Box No 1**
**B-1150 Brussels (BE)**

## Description

The present invention relates to 2-hydroxy-4-methylthiobutanoic acid, an analogue of the essential amino acid methionine, commonly referred to as methionine hydroxy analogue. More particularly, the present invention relates to solid methionine hydroxy analogue compositions which have a substantially higher 2-hydroxy-4-methylthiobutanoic acid content than solid compositions reported heretofore.

Various methods for preparing methionine are known. For example, the synthesis of methionine from methyl mercaptan and acrolein involves the preparation of methylmercaptopropionaldehyde which is reacted with hydrocyanic acid to form 2-hydroxy-4-methylthiobutyronitrile which is subsequently aminated and hydrolyzed to methionine. Unfortunately, the reaction of 2-hydroxy-4-methylthiobutyronitrile with ammonia is a difficult and costly procedure requiring the use of high pressure apparatus. It was subsequently found that methionine analogues such as 2-hydroxy-4-methylthiobutanoic acid, hereinafter referred to as HMBA, are virtually as effective as methionine for nutritional uses, particularly as a poultry feed supplement. However, the crystalline form of HMBA has a melting point between 38 °C and 42 °C depending on the purity, rendering it susceptible to liquid-solid phase change upon storage or use at ambient temperatures. Since all feed milling operations, particularly those in developing countries, are not equipped to handle liquids in their mixing processes, a stable, solid form of HMBA is often more practical.

A solid form of HMBA has been disclosed wherein the acid is sprayed onto a solid support such as feed grade vermiculite, for example see Romoser et al., Poult. Sci. *55*, p. 1099-1103 (1976). Unfortunately, at the high loadings necessary to achieve reasonable methionine activity per unit weight the composition often becomes sticky, does not flow freely and is highly hygroscopic.

While various salts of 2-hydroxy-4-methylthiobutanoic acid have been disclosed, only the calcium salt of HMBA is approved by the United States Food and Drup Administration for use as an animal feed supplement. The conventional calcium salt of HMBA, hereinafter referred to as Ca(HMBA)$_2$, consists of two moles of HMBA equivalent per mole of calcium. Moreover, methods disclosed for preparing the above-described calcium salt teach reacting essentially stoichiometric amounts of 2-hydroxy-4-methylthiobutanoic acid and calcium oxide or calcium hydroxyde to yield the conventional ionic salt, for example see U.S. patent 4,335,257.

It is therefore the overall object of the present invention to provide a solid form of HMBA having substantially higher HMBA equivalent per unit weight than solid forms disclosed heretofore.

It is an object of the present invention to provide a solid form of HBMA which flows freely and is not highly hygroscopic.

It is another object of the present invention to provide a calcium salt complex of HMBA having substantially higher HMBA equivalent than the conventional calcium salt disclosed heretofore.

It is yet another object of the present invention to provide a process for preparing the enhanced HMBA compositions embraced by the present invention.

These and other objects, features and advantages of the present invention will become evident to those skilled in the art from the following description.

## Summary of the invention

The present invention provides an enhanced 2-hydroxy-4-methylthiobutanoic acid composition comprising greater than two and less than about ten moles of HMBA equivalent per mole of calcium. Compositions comprising less than about six moles of HMBA equivalent per mole of calcium are preferred since these compositions made by the process of this invention are free flowing powders or agglomerates and therefore possess superior blending characteristics.

The composition can be easily prepared by contacting additional HMBA with the conventional calcium salt or by contacting calcium oxide, hydroxide or carbonate with HMBA in the presence or absence of a heel of the conventional calcium salt of HMBA or the enhanced HMBA composition of the present invention. Alternately, compositions comprising greater than two and less than about four moles of HMBA equivalent per mole or calcium can be easily formed by crystallization from an aqueous solution of HBMA and Ca(HMBA)$_2$, calcium oxide or calcium hydroxide.

## Detailed description of the invention

It has been found that when the calcium salt of 2-hydroxy-4-methylbutanoic acid is prepared in the presence of an excess of HMBA a salt complex is formed. The salt complex has a distinctly different crystalline structure than Ca(HMBA)$_2$ as evidenced by X-ray powder diffraction. While not fully understood, the salt complex embraced by the present invention comprises about four moles of HBMA per mole of calcium and has a melting point of about 130 °C.

While Ca(HBMA)$_2$ consists of approximately 88.8 wt% HMBA equivalent, the pure salt complex of the present invention consists of approximately 94.0 wt% HMBA equivalent. It should therefore be evident to one skilled in the art that preparation and use of the enhanced HMBA compositions of the present

2

invention affords numerous advantages over the conventional calcium salt as a methionine activity source in animal rations. The enhanced compositions have greater methionine activity per unit weight, lower manufacturing cost per unit methionine activity and requires less storage space per unit methionine activity.

The usual commercial form of HMBA is the optically inactive, racemic D,L-HMBA mixture. It should be understood that while the HMBA compositions referred to hereinafter are racemic mixtures, the individual D- und L-isomers of HMBA can be converted to the enhanced solid HMBA compositions of the present inventions by the procedures described hereinafter. Hence, for purposes of the present invention by « HMBA » is meant either the D- or L-isomer of 2-hydroxy-4-methylthiobutanoic acid or any mixture of the two above-described isomers thereof.

While the present invention embraces the formation of the above-described salt complex, the present invention contemplates in its broadest aspects the discovery that enhanced HMBA compositions can be prepared in solid form which comprise greater than two moles and less than about ten moles of HMBA equivalent per mole of calcium. Moreover, HMBA is present in essentially monomeric form. When more than four moles of HMBA are used per mole of calcium, the excess free HMBA is believed to be trapped as inclusions in the agglomerates of the salt complex crystals. While not fully understood, compositions comprising less than four moles of HMBA equivalent per mole of calcium are believed to exist as a mixture of conventional calcium salt made up of 2 moles of HMBA equivalent per mole of calcium and the above-described complex. Compositions comprising a mixture of $Ca(HMBA)_2$ and enhanced salt complex only partially melt at the salt complex melting temperature of about 130 °C. Compositions comprising less than about six moles of HMBA equivalent per mole of calcium are preferred since these compositions prepared by the process described hereinafter are free flowing powders or agglomerates and therefore possess superior blending characteristics.

By « HMBA equivalent » is meant the amount of free HMBA which corresponds to the HMBA present in the composition whether in free acid or ionized form. For example, while not fully understood, compositions comprising four moles of HMBA equivalent per mole of calcium are believed to comprise two moles of free acid and two moles of ionized HMBA per mole of calcium.

The enhanced HMBA compositions of the present invention are preferably prepared by contacting the conventional calcium salt with additional HMBA. If necessary, water can be easily removed by heating the enhanced HMBA compositions above about 70 °C. When two moles of HMBA are contacted with one mole of $Ca(HMBA)_2$, a highly crystalline complex is formed. When more than two moles of HMBA are contacted with one mole of $Ca(HMBA)_2$ ·agglomerates of the complex are formed. The agglomerates increase in size as the molar ratio of HMBA to calcium increases above four.

Compositions of the present invention can also be prepared by contacting one mole of calcium oxide, hydroxide or carbonate with more than two moles of HMBA (neat or in aqueous solution) in the presence or absence of a heel of the conventional calcium salt of HMBA or the enhanced salt complex of the present invention. While it should be understood that the presence of a heel of the enhanced salt complex or $Ca(HMBA)_2$ is not critical to the present invention, the presence of a heel renders the mixture less sticky and easier to admix to substantial homogeneity. If a heel is used, it is preferred that the heel comprise at least 20 percent of the total mass of the mixture. It should be further understood that while temperature is not a critical parameter to the present invention, temperatures above about 70 °C are preferred to obtain a powdery or granular product.

Finally, enhanced HMBA compositions comprising greater than two and less than about four moles of HMBA equivalent per mole of calcium can be crystallized from an aqueous solution comprising HMBA and $Ca(HMBA)_2$, calcium oxide or calcium hydroxide. For example, the enhanced salt complex comprising about four moles of HMBA equivalent per mole of calcium can be crystallized from an aqueous solution comprising between about 2.5 and 3.0 moles of HMBA per mole of $Ca(HMBA)_2$. The reactants are preferably dissolved at a temperature above about 70 °C and then slowly cooled to room temperature. Drying the product composition at temperatures above about 70 °C frees the water of hydration and produces essentially fine crystals. Analysis of the salt complex composition crystallized indicates that the free HMBA included in the salt complex is essentially in monomeric form.

The following examples are included to better illustrate the practice of the present invention. It should be understood that these examples are included for illustrative purposes only and are not, in any way, intended to limit the scope of the present invention. While preparation of the enhanced HMBA compositions is described hereinafter with reference to batch operations, it is evident to those skilled in the art that continuous processing is also suitable for practice of the present invention. Although the aqueous solutions used hereinafter are fairly concentrated, the amount of water present in the process of the present invention should not be considered critical, but rather a matter of economics since water remaining in wet products must be evaporated off to obtain a free flowing, powdery product. Unless otherwise noted, all percentages are by weight and all temperatures are in degrees centigrade.

Example 1

A batch operation was performed in a laboratory-scale double arm, sigma blade mixer. The mixing chamber was open to the atmosphere in a laboratory hood. A 372.3 gm sample of $Ca(HMBA)_2$ was

warmed to 85 °C with low pressure steam in the jacket of the mixer. Four portions (93.3 gm, 92.6 gm, 94.5 gm and 96.3 gm) of an aqueous 88 % HMBA solution were uniformly added to the mixer from a beaker at about fifteen minute intervals. The composition was almost dry within fifteen minutes of each HMBA addition. The composition was further mixed and dried in the laboratory mixer for twenty-seven minutes at 73°-82 °C following the last HMBA addition. A 634.8 gm mass of product was recovered from the mixer. Subsequent loss on drying at 70 °C overnight was approximately 0.4 wt%. Analysis of the dry powdery product by X-Ray powder diffraction showed a highly crystalline structure which was distinctly different from Ca(HMBA)$_2$. The enhanced salt complex and Ca(HMBA)$_2$ content of the dry product were estimated to be greater than 90 wt% and less than 10 wt%, respectively by X-Ray powder diffraction and gas chromatographic determination of the free HMBA monomer content of the product. The product composition melted at about 130 °C.

## Example 2

A 284.5 gm heel of Ca(HMBA)$_2$ and 27.8 gms of calcium hydroxide were mixed and warmed to 78 °C as described in Example 1. A 256.1 gm sample of an aqueous 88 % HMBA solution as described above was added to the mixer chamber over a five minute period at 78°-86 °C. The resulting composition was mixed and dried in the mixer at 74°-82 °C for about thirty-seven minutes. The partially dried product was further dried overnight at 70 °C.

Analysis of the dried product indicated an HMBA equivalent of 91.8 wt%. The enhanced salt complex and Ca(HMBA)$_2$ content of the dry product were estimated to be about 42 wt% and 58 wt%, respectively, by X-Ray powder diffraction and gas chromatographic determination of the free HMBA monomer content of the product. The product partially melted at about 130°.

## Example 3

Calcium hydroxide and an aqueous 88 % HMBA solution were mixed in various proportions in evaporating dishes at room temperature (22 °C) using a metal spatula. The product compositions were either wet powder, dough-like material, or paste depending on the reactant proportions. After being dried overnight at 70 °C and then ground all product compositions became free flowing powders. Analytical results of product compositions comprising greater than two and up to about four moles of HMBA equivalent per mole of calcium are summarized as follows :

| Composition | I | II | III | IV | V | VI |
|---|---|---|---|---|---|---|
| 88% HMBA, gm. | 16.8 | 16.8 | 16.8 | 16.8 | 16.8 | 16.8 |
| Ca(OH)$_2$, gm. | 3.7 | 3.3 | 3.0 | 2.6 | 2.2 | 1.8 |
| Product, wt% | | | | | | |
| HMBA equiv. | 86.9 | 87.1 | 89.3 | 90.8 | 90.6 | 93.2 |
| Water | 0.0 | 2.2 | 2.5 | 1.2 | 0.9 | 1.5 |

It should be noted from the results listed in the above table that compositions comprising salt complex and Ca(HMBA)$_2$ prepared at low temperatures (22°-70 °C) tend to form hydrates. It should be further noted, however, that the hydrates may be freed of water of hydration by drying the compositions at temperatures above about 70 °C. All of the above product compositions partially melted between 121 °C and 132 °C.

## Example 4

A 34.1 gm sample of an aqueous 88 % HMBA solution was mixed with 3.71 gms of calcium hydroxide in an evaporating dish at room temperature (22 °C) and dried overnight at 22 °C. When the moisture content had reached 11 wt%, the product composition was extruded through 16 mesh metal screen to produce a granular product. After drying onvernight at 70 °C the extrudate was a hard, dried spaghetti-like material. The total HMBA equivalent content was 94.7 wt%. The product partially melted at about 131 °C.

## Example 5

A 34.1 gm sample of an aqueous 88 % HMBA solution was mixed with 2.80 gms of calcium oxide in an evaporating dish at room temperature (22 °C) and dried overnight 22 °C. When the moisture content had reached 10 wt%, the product composition was extruded through a 16 mesh metal screen to produce a granular product. After drying overnight at 70 °C, the extrudate was a hard, dried spaghetti-like material. Total HMBA equivalent content was 95.3 wt%. The product partially melted at about 130 °C.

### Example 6

A 92.4 gm sample of Ca (HMBA)$_2$ (HMBA equivalent 87.09 wt %) and 114.6 gms of an aqueous 88 % HMBA solution were mixed in 343.8 gms of water and warmed to 92 °C. The solid particles were filtered out and the filtrate permitted to cool. Upon slow cooling overnight at room temperature (22 °C) the filtrate yielded a large quantity of precipitate. Analysis of product crystals after drying overnight at 70 °C indicated a total HMBA equivalent of 93.5 wt%. The dried product was a free flowing powder.

Under microscopic examination, the crystals of salt complex appeared as shiny glassy material while conventional Ca(HMBA)$_2$ crystals appeared as white powder.

### Example 7

A 156.9 gm heel of Ca(HMBA)$_2$ and 28.9 gms of calcium oxide were mixed and warmed to 88 °C in a laboratory-scale double arm, sigma blade mixer. A 337.6 gm aliquot of an aqueous 89 wt% HMBA solution was slowly added to the mixer chamber over a seventy-three minute period. Moisture was evaporated off over an additional eighteen minute period.

Analysis of the product without further drying indicated an HMBA equivalent content of 92.4 wt%. The product melted at about 128 °C.

### Example 8

A 113.5 gm heel of Ca(HMBA)$_2$ and 25.5 gms of calcium hydroxide were mixed and warmed to 81 °C in a preheated laboratory-scale double arm, sigma blade mixer. A 428.5 gm sample of an aqueous 70 wt% HMBA solution was slowly added to the mixer chamber over a one hundred thirty-six minute period. Moisture was evaporated off over an additional twenty-five minute period.

Analysis of the product without further drying indicated an HMBA equivalent content of 94.0 wt%. The product melted at about 127 °C.

### Example 9

A 180.4 gm aliquot of a 98 wt% HMBA solution was slowly added to 200.0 gm sample of Ca(HMBA)$_2$ in a laboratory-scale planetary mixer at 25 °C.

Analysis of the undried product indicated a HMBA equivalent content of 92.1 wt%. The product melted at about 119 °C.

### Example 10

A 38.2 gm sample of calcium hydroxide was mixed with a 93.8 gm heel of enhanced salt complex comprising about four moles of HMBA equivalent per mole of calcium in a laboratory-scale sigma blade mixer preheated to 72 °C. A 338.2 gm sample of an aqueous 89 wt% HMBA solution was slowly added to the mixture over a seventy-five minute period. Moisture was evaporated off over an additional fifteen minute period.

Analysis of the product without further drying indicated an HMBA equivalent content of 94.3 wt%. The product melted at about 125 °C.

### Example 11

Calcium hydroxide was mixed with varying concentrations of an aqueous HMBA solution so that in each case calcium and HMBA were present in a one to four molar ratio. The HMBA solutions used ranged from 50.9 to 99.8 wt% HMBA. The compositions were mixed in evaporating dishes at room temperature (22 °C) using a metal spatula. Products were either dough-like material or paste depending on the amount of water involved. However, after being dried overnight at 70 °C and then ground all product compositions were free flowing powders. All dried product compositions exhibited an angle of repose of approximately 40 degrees.

### Example 12

Calcium hydroxide and an aqueous 88 wt% HMBA solution were mixed in various proportions so as to attain mixtures corresponding to between 4 and 10 moles (at integral increments, inclusive) of HMBA equivalent per mole of calcium. The compositions were mixed in evaporating dishes at room temperature (22 °C) using a metal spatula. The product compositions varied from dough-like material to paste depending on reactant concentrations. After being dried overnight at 70 °C and then ground, compositions comprising four to six moles of HMBA equivalent per mole of calcium were free flowing powders with an angle of repose of about 40 degrees. Compositions comprising seven to ten moles of HMBA equivalent per mole of calcium were tacky to sticky materials with an angle of repose between about fifty and eighty-five degrees.

## Claims

1. A solid calcium-HMBA composition which comprises greater than two and less than ten moles of 2-hydroxy-4-methylthiobutanoic acid equivalent per mole of calcium in said composition.

2. The composition of Claim 1 in which said acid content is greater than two and less than six moles of 2-hydroxy-4-methylthiobutanoic acid equivalent per mole of calcium.

3. The composition of Claim 1 in which said acid content is greater than four and less than six moles of 2-hydroxy-4-methylthiobutanoic acid equivalent per mole of calcium.

4. The composition of Claim 1 in which said acid content is about four moles of 2-hydroxy-4-methylthiobutanoic acid equivalent per mole of calcium.

5. A method of preparing an enhanced 2-hydroxy-4-methylthiobutanoic acid composition which comprises contacting 2-hydroxy-4-methylthiobutanoic acid with the conventional calcium salt of 2-hydroxy-4-methylthiobutanoic acid so that the overall content of the mixture comprises greater than two and less than ten moles of 2-hydroxy-4-methylthiobutanoic acid equivalent per mole of calcium.

6. A method of preparing an enhanced 2-hydroxy-4-methylthiobutanoic acid composition which comprises contacting 2-hydroxy-4-methylthiobutanoic acid with the composition of Claim 4 so that the overall content of the mixture comprises greater than two and less than ten moles of 2-hydroxy-4-methylthiobutanoic acid equivalent per mole of calcium.

7. A method of preparing an enhanced 2-hydroxy-4-methylthiobutanoic acid composition which comprises contacting 2-hydroxy-4-methylthiobutanoic acid with a calcium source selected from calcium oxide, calcium hydroxide and calcium carbonate so that the overall content of the mixture comprises greater than two and less than ten moles of 2-hydroxy-4-methylthiobutanoic acid equivalent per mole of calcium.

8. The method of Claim 7 in which the 2-hydroxy-4-methylthiobutanoic acid is contacted with said calcium source and with the conventional calcium salt of 2-hydroxy-4-methylthiobutanoic acid.

9. The method of Claim 8 in which the conventional salt of 2-hydroxy-4-methylthiobutanoic acid constitutes at least twenty percent of the total mass of the mixture.

10. The method of Claim 7 in which the 2-hydroxy-4-methylthiobutanoic acid is contacted with said calcium source and with the composition of Claim 4.

11. The method of Claim 10 in which the composition of Claim 4 constitutes at least twenty percent of the total mass of the mixture.

12. The method of any of Claims 7 to 11 in which the contacting is carried out at a temperature of at least 70 °C.

## Patentansprüche

1. Feste Calcium-HMBA-Zusammensetzung enthaltend mehr als 2 und weniger als 10 Mol 2-Hydroxy-4-methylthiobutansäure-Äquivalente pro Mol Calcium in der Zusammensetzung.

2. Zusammensetzung nach Anspruch 1, dadurch gekennzeichnet, daß der Säuregehalt mehr als 2 und weniger als 6 Mol 2-Hydroxy-4-methylthiobutansäure-Äquivalente pro Mol Calcium beträgt.

3. Zusammensetzung nach Anspruch 1, dadurch gekennzeichnet, daß der Säuregehalt mehr als 4 und weniger als 6 Mol 2-Hydroxy-4-methylthiobutansäure-Äquivalente pro Mol Calcium beträgt.

4. Zusammensetzung nach Anspruch 1, dadurch gekennzeichnet, daß der Säuregehalt etwa 4 Mol 2-Hydroxy-4-methylthiobutansäure-Äquivalente pro Mol Calcium beträgt.

5. Verfahren zur Herstellung einer angereicherten 2-Hydroxy-4-methylthiobutansäure-Zusammensetzung, dadurch gekennzeichnet, daß man 2-Hydroxy-4-methylthiobutansäure mit dem üblichen Calciumsalz von 2-Hydroxy-4-methylthiobutansäure derart in Kontakt bringt, daß die gesamte Mischung mehr als 2 und weniger als 10 Mol 2-Hydroxy-4-methylthiobutansäure-Äquivalent pro Mol Calcium enthält.

6. Verfahren zur Herstellung einer angereicherten 2-Hydroxy-4-methylthiobutansäure-Zusammensetzung, dadurch gekennzeichnet, daß man 2-Hydroxy-4-methylthiobutansäure mit der Zusammensetzung des Anspruchs 4 derart in Kontakt bringt, daß der Gesamtgehalt der Mischung mehr als 2 und weniger als 10 Mol 2-Hydroxy-4-methylthiobutansäure-Äquivalente pro Mol Calcium ausmacht.

7. Verfahren zur Herstellung einer angereicherten 2-Hydroxy-4-methylthiobutansäure-Zusammensetzung, dadurch gekennzeichnet, daß man 2-Hydroxy-4-methylthiobutansäure mit einer aus Calciumoxid, Calciumhydroxid und Calciumcarbonat ausgewählten Calciumquelle derart in Kontakt bringt, daß der Gesamtgehalt der Mischung mehr als 2 und weniger 10 Mol 2-Hydroxy-4-methylthiobutansäure-Äquivalente pro Mol Calcium beträgt.

8. Verfahren nach Anspruch 7, dadurch gekennzeichnet, daß die 2-Hydroxy-4-methylthiobutansäure mit der Calciumquelle und mit dem üblichen Calciumsalz der 2-Hydroxy-4-methylthiobutansäure in Kontakt gebracht wird.

9. Verfahren nach Anspruch 8, dadurch gekennzeichnet, daß das übliche Salz der 2-Hydroxy-4-methylthiobutansäure mindestens 20 % der Gesamtmasse der Mischung ausmacht.

10. Verfahren nach Anspruch 7, dadurch gekennzeichnet, daß die 2-Hydroxy-4-methylthiobutansäure mit der Calciumquelle und mit der Zusammensetzung des Anspruchs 4 in Kontakt gebracht wird.

11. Verfahren nach Anspruch 10, dadurch gekennzeichnet, daß die Zusammensetzung des Anspruchs 4 mindestens 20 % der Gesamtmasse der Mischung ausmacht.

12. Verfahren nach einem der Ansprüche 7 bis 11, dadurch gekennzeichnet, daß das Inkontaktbringen bei einer Temperatur von mindestens 70 °C bewirkt wird.

**Revendications**

1. Composition solide calcium-HMBA qui comprend plus de deux et moins de dix moles d'équivalent d'acide 2-hydroxy-4-méthylthiobutanoïque par mole de calcium dans cette composition.

2. Composition de la revendication 1, dans laquelle cette teneur en acide est supérieure à deux et inférieure à six moles d'équivalent acide 2-hydroxy-4-méthylthiobutanoïque par mole de calcium.

3. Composition de la revendication 1, dans laquelle cette teneur en acide est supérieure à quatre et inférieure à six moles d'équivalent d'acide 2-hydroxy-4-méthylthiobutanoïque par mole de calcium.

4. Composition de la revendication 1, dans laquelle cette teneur en acide est d'environ quatre moles d'équivalent d'acide 2-hydroxy-4-méthylthiobutanoïque par mole de calcium.

5. Procédé de préparation d'une composition d'acide 2-hydroxy-4-méthylthiobutanoïque améliorée, qui consiste à mettre en contact de l'acide 2-hydroxy-4-méthylthiobutanoïque avec le sel de calcium classique de l'acide 2-hydroxy-4-méthylthiobutanoïque de telle sorte que la teneur globale du mélange contienne plus de deux et moins de dix moles d'équivalent d'acide 2-hydroxy-4-méthylthiobutanoïque par mole de calcium.

6. Procédé de préparation d'une composition d'acide 2-hydroxy-4-méthylthiobutanoïque améliorée, qui consiste à mettre en contact de l'acide 2-hydroxy-4-méthylthiobutanoïque avec la composition de la revendication 4, de telle sorte que la teneur globale du mélange contienne plus de deux et moins de dix moles d'équivalent d'acide 2-hydroxy-4-méthylthiobutanoïque par mole de calcium.

7. Procédé de préparation d'une composition d'acide 2-hydroxy-4-méthylthiobutanoïque améliorée, qui consiste à mettre en contact de l'acide 2-hydroxy-4-méthylthiobutanoïque avec une source de calcium choisie parmi l'oxyde de calcium, l'hydroxyde de calcium et le carbonate de calcium, de telle sorte que la teneur globale du mélange contienne plus de deux et moins de dix moles d'équivalent d'acide 2-hydroxy-4-méthylthiobutanoïque par mole de calcium.

8. Procédé de la revendication 7, dans lequel l'acide 2-hydroxy-4-méthylthiobutanoïque est mis en contact avec cette source de calcium et avec le sel de calcium classique de l'acide 2-hydroxy-4-méthylthiobutanoïque.

9. Procédé de la revendication 8, dans lequel le sel classique de l'acide 2-hydroxy-4-méthylthiobutanoïque constitue au moins vingt pour cent de la masse totale du mélange.

10. Procédé de la revendication 7, dans lequel de l'acide 2-hydroxy-4-méthylthiobutanoïque est mis en contact avec cette source de calcium et avec la composition de la revendication 4.

11. Procédé de la revendication 10, dans lequel la composition de la revendication 4 constitue au moins vingt pour cent de la masse totale du mélange.

12. Procédé suivant l'une quelconque des revendications 7 à 11, dans lequel la mise en contact est effectuée à une température d'au moins 70 °C.